# EUROPEAN PATENT APPLICATION

(11) **EP 1 982 728 A1**
(43) Date of publication of application: **22.10.2008**
(21) Application number: 07707230.4
(22) Date of filing: 23.01.2007
(51) Int. Cl.: A61K 39/29, A61P 1/16, A61P 31/14, C07K 7/06, G01N 33/576

(54) **PEPTIDE DERIVED FROM HEPATITIS C VIRUS**

(30) Priority: 23.01.2006 JP 2006014000; 11.08.2006 JP 2006220542
(71) Applicant: Green Peptide Co., Ltd., Kurume-shi, Fukuoka 8300018 (JP)
(72) Inventor: YAMADA, Akira, Ogori-shi, Fukuoka 838-0103 (JP); ITOH, Kyogo, Miyaki-gun, Saga 841-0205 (JP); SATA, Michio, Fukuoka-shi, Fukuoka 814-0002 (JP); YUTANI, Shigeru, Kurume-shi, Fukuoka 830-0022 (JP)
(74) Representative: Vossius & Partner
(86) International application number: PCT/JP2007/050967
(87) International publication number: WO 2007/083807

(57) **Abstract**

Disclosed is a pharmaceutical composition for the treatment of an HCV-related disease in a patient having HLA-A24 or -A3 supertype, wherein the pharmaceutical composition comprises a peptide having the amino acid sequence: YLLPRRGPRL (SEQ ID NO:1) as an active ingredient. Also disclosed is a composition for use in the detection of the induction of an HLA-A24 or -A3 supertype-restricted cytotoxic T cell in a patient having HLA-A24 or -A3 supertype, wherein the composition comprises a peptide having the amino acid sequence depicted in SEQ ID NO:1 as an active ingredient.

SEQUENCE LISTING

<110> GREEN PEPTIDE CO., LTD.

<120> Peptide derived from Hepatitis C virus

<130> P2797 EP

<140> 07 70 7230.4
<141> 2007-01-23

<150> JP 2006-220542
<151> 2006-08-11

<150> JP 2006-14000
<151> 2006-01-23

<160> 8

<170> PatentIn version 3.1

<210> 1
<211> 10
<212> PRT
<213> Hepatitis C Virus

<400> 1
Tyr Leu Leu Pro Arg Arg Gly Pro Arg Leu
1
5 10
<210> 2
<211> 10
<212> PRT
<213> Influenza Virus

<400> 2
Arg Phe Tyr Ile Gln Met Cys Thr Glu Leu
1               5                   10

<210> 3
<211> 9
<212> PRT
<213> Epstein-Barr Virus

<400> 3
Thr Tyr Gly Pro Val Phe Met Cys Leu
1               5

<210> 4
<211> 9
<212> PRT
<213> Human Immunodeficiency Virus

<400> 4
Arg Tyr Leu Arg Asp Gln Gln Leu Leu
1               5

<210> 5
<211> 10
<212> PRT
<213> Influenza virus

<400> 5
Asn Val Lys Asn Leu Tyr Glu Lys Val Lys
1               5                   10

<210> 6
<211> 10

<212>  PRT
<213>  Epstein Barr virus

<400>  6
Ala Val Phe Asp Arg Lys Ser Asp Ala Lys
1               5                   10

<210>  7
<211>  9
<212>  PRT
<213>  Homo sapiens

<400>  7
Leu Leu Gly Pro Gly Arg Pro Tyr Arg
1               5

<210>  8
<211>  11
<212>  PRT
<213>  Human immunodeficiency virus

<400>  8
Arg Leu Arg Asp Leu Leu Leu Ile Val Thr Arg
1               5                   10

## Description

### TECHNICAL FIELD

The present invention relates to the treatment of a hepatitis C virus-related disease.

### BACKGROUND

The hepatitis C virus (HCV) is a single-stranded RNA virus belonging to Flaviviridae, and approximately 3% of the world population is estimated to be HCV-seropositive (World Health Organization. Hepatitis C: global prevalence. Wkly. Epidemiol. Rec. 1997; 72(46):341-344). The acute phase of HCV infection is often asymptomatic, but chronic infections occur in approximately 70% of seropositive individuals, and infected individuals are susceptible to progressive liver disease, e.g., chronic hepatitis, hepatocirrhosis, and hepatocarcinoma (Alter HJ, Seeff LB., Semin. Liver Dis. 2000; 20 (1) :17-35; Shimotohno, K., Semin. Cancer Biol. 2000;10:233-240). The present method of treatment for HCV infections is a combination of ribavirin and interferon (IFN)-a, and recently IFN-a modified with polyethylene glycol has been used. However, a sustained response is seen in only about 50% of treated patients (Manns MP, et al, Lancet. 2001; 358:958-965). Even more importantly, antiviral therapy cannot be used in almost all of the developing countries where the majority of infected patients reside. Therefore, development of a new method of treatment is needed.

The pathogenic mechanism of damage to hepatocytes subsequent to HCV infection is still not well understood, but it has been shown by a preponderance of evidence that virus-specific cytotoxic T lymphocytes (CTL) play an important role in liver damage after HCV infection (Chang KM, et al., Springer Semin Immunopathol. 1997; 19:57-68). On the other hand, CTL are thought to be effective in restricting the spread of viruses after infection and eliminating them (Kurokohchi K, et al., J Virol. 1996; 70:232-240). Therefore, CTL induction by a vaccine appears to be a potential strategy for managing diseases associated with HCV infection. In addition, the development of a peptide vaccine for inducing CTL is desirable because of low cost and ease of storage.

The inventors have previously discovered that IgG that reacts with an HCV-derived peptide can be detected in most HCV-infected patients, and that peptide-specific CTL having HLA types matching those peptides can be induced from the peripheral blood monocytes (PBMC) of HCV patients. In addition, the inventors have discovered that when an HCV-derived peptide is administered, an immunostimulatory effect and a decrease in the amount of virus caused by the peptide are seen in most of the HLA-A2 patients (Takao Y et al., Microbiol. Immunol., 48(7), 507-517, 2004 [Non-patent document 1], WO2005/028503 [Patent document 1], Japanese Patent Application 2005-310203 [Patent document 2]).

However, because there are patients who cannot induce CTL against these peptides, an additional and distinct peptide that may be used in such patients is needed in the art. More specifically, HLA-A24 is seen second most frequently after HLA-A2, and therefore a peptide that can be used in HLA-A24 patients is needed. On the other hand, HLA-A11, HLA-A31, and HLA-A33, as well as HLA-A0301 and HLA-A6801 all share a similar antigen binding motif, and therefore are classified together as HLA-A3 supertype (Non-patent document 2). An HLA-A3 supertype-restricted antigen peptide derived from HCV has not been reported until now.

Reference documents cited in the specification are listed below. The contents of these documents are hereby incorporated by reference in its entirety. None of these documents is admitted to be prior art with respect to the present invention.
[Patent document 1] WO2005/028503
[Patent document 2] Japanese Patent Application 2005-310203
[Non-patent document 1] Takao Y et al., Microbiol. Immunol., 48(7), 507-517, 2004
[Non-patent document 2] Takedatsu H, et al., Clin Cancer Res 2004; 10: 1112-1120

### DISCLOSURE OF THE INVENTION

An object of the present invention is to provide a method for treatment of an HCV-related disease that can be used in HCV-infected patients with HLA (Human Leukocyte Antigen)-A24 and -A3 supertype.

The present invention provides a pharmaceutical composition for the treatment of an HCV-related disease in a patient having HLA-A24 or -A3 supertype comprising a peptide having an amino acid sequence represented by the following sequence YLLPRRGPRL (SEQ ID NO: 1) as an active ingredient. The present invention also provides a composition for detecting the induction of an HLA-A24 or -A3 supertype-restricted cytotoxic T cell in a patient having HLA-A24 or -A3 supertype, respectively, comprising a peptide having an amino acid sequence represented by SEQ ID NO: 1 as an active ingredient.

As used herein, the term "HCV-related disease" refers to a disease brought about by infection by the hepatitis C virus, including, for example, acute and chronic hepatitis, hepatocirrhosis, and hepatocarcinoma. The progression of an HCV-related disease typically goes from chronic hepatitis to hepatocirrhosis, and then from hepatocirrhosis to hepatocarcinoma.

The peptide of the present invention (YLLPRRGPRL (SEQ ID NO: 1)) is derived from the sequence of the HCV core protein, and is also referred to "peptide C 35-44" herein. It has been reported that this peptide can strongly induce cytotoxic T lymphocytes (CTL) that are effective in eliminating viruses from the peripheral blood of HLA-A2 positive individuals. The inventors have surprisingly discovered that the peptide C 35-44 of the present invention can induce CTL in patients that are HLA-A24 or -A3 supertype positive even though it has been shown to have extremely low binding activity with HLA-A24 or -A11 when the dissociation constants therewith are calculated by the conventional method. A vaccine targeting HLA-A2 positive patients may cover only about 40% of Japanese patients. It has now clearly demonstrated that the peptide vaccine of the invention can be used for both HLA-A24 positive and -A3 supertype positive patients, which means that almost 100% of Japanese patients can be targeted by the peptide vaccine therapy of the present invention. Therefore, the peptide of the present invention can be used even when the HLA type of the patient and antibody titer in the serum of the patient are unknown. Furthermore, the peptide C 35-44 can be used for many patients of HCV infection because the peptide sequence is widely conserved among various HCV genotypes such as HCV-1b, HCV-2a, and the like.

### BRIEF DESCRIPTION OF THE DRAWINGS

FIG. 1 shows the cytotoxic activity of peptide-stimulated PBMC on cells previously loaded with the peptide;
FIG. 2 shows the cytotoxic activity of peptide-stimulated PBMC on cells previously loaded with the peptide;
FIG. 3 shows a comparison of the HLA binding capability of various peptides;
FIG. 4 shows the binding capability of the peptide C35-44 to HLA-A3 supertype; and
FIG. 5 shows the MHC restriction of CTL induced by the peptide C35-44.

### PREFERRED EMBODIMENT OF THE INVENTION

The present invention provides a vaccine containing, as an active ingredient a peptide derived from immunogenic HCV, which can be used for the treatment of HCV-infected patients having the following HLA types: HLA-A24, HLA-A11, HLA-A31, and HLA-A33. As described in the examples below, it has now been discovered that the peptide C35-44 induces peptide specific CTL from peripheral blood monocytes (PBMC) of HLA-A24-positive HCV-infected individuals. In addition, binding experiments with MHC molecules demonstrated that the peptide C35-44 binds to HLA-A24. Furthermore, IgG antibodies that react with the peptide C35-44 were detected in the plasma of HLA-A11, HLA-A31 and HLA-A33 positive HCV-infected patients suffering from an HCV-related disease. Moreover, the peptide of the present invention induces peptide-specific CTL from PBMC of HLA-A31 and -A33 positive HCV-infected individuals.

The peptide derived from the hepatitis C virus (HCV peptide) of the present invention comprises the partial amino acid sequence of a protein derived from the hepatitis C virus, and it can be recognized by antibodies in the blood of patients infected with the hepatitis C virus. Preferably, the peptide derived from the hepatitis C virus of the present invention can induce CTL, and the CTL in tern will target and attack HCV-infected cells.

The peptide of the present invention may also contain an additional sequence to the aforementioned amino acid sequence such that it provides the desired effect. A person skilled in the art readily understands the extent of sequence addition to the N-terminus and C-terminus that will be acceptable to provide the desired effect as an antigen peptide. Therefore, examples of an amino acid sequence that can be added to the N-terminus and/or C-terminus of the peptide of the present invention include a sequence useful for promoting immunization, a sequence that facilitates formulating the peptide into a drug product, a sequence useful for expressing the peptide as a fusion protein, a sequence useful for production and purification of peptides, and the like. Furthermore, the peptide of the present invention may be chemically modified, and polymers and sugar chains may be added thereto as long as the antibody binding specificity is not lost.

The HCV peptide of the present invention can be produced by conventional chemical synthesis, enzymatic degradation of a protein molecule, gene recombination technology utilizing a transformed host that expresses a nucleotide sequence encoding the amino acid sequence of interest.

The peptide of the invention may be produced by chemical synthesis using well known and commonly practiced means in conventional peptide chemistry by, for example, utilizing a peptide synthesis apparatus and synthesizing the peptide via solid phase synthesis. The crude peptide obtained thereby can be purified by a purification method conventionally used in protein chemistry, for example, salting out, ultrafiltration, reverse phase chromatography, ion exchange chromatography, affinity chromatography, and the like.

On the other hand, the peptide of the invention can be produced by gene recombination technology by incorporating a synthesized or cloned DNA fragment encoding the desired amino acid sequence into a suitable expression vector, transforming a microorganism or animal cell using that expression vector, and culturing the transformant obtained thereby. A plasmid or a virus vector that is well known in the art can be used as the expression vector.

When an expression vector is used in the peptide production, any of known methods may be used for transforming host cells, including, for example, calcium chloride method, calcium phosphate coprecipitation method, DEAE dextran method, the lipofectin method, electroporation method, and the like, which can be suitably selected based on the host cells used. The peptide obtained thereby can be purified by the purification methods described above from an extract of cells collected from the culture medium or from the culture supernatant.

The peptide of the present invention is useful as a pharmaceutical composition, i.e., as a peptide vaccine, for treating patients with an HCV-related disease. The vaccine comprising the peptide derived from the hepatitis C virus can be prepared by suitably admixing a peptide prepared as described above with a pharmaceutically acceptable adjuvant and/or vehicle. Any adjuvants that will potentiate the immune response can be used as the adjuvant, including, for example, incomplete Freund's adjuvant, aluminum hydroxide gel, and the like. Examples of a vehicle that can be used include phosphate-buffered saline (PBS), a diluent such as distilled water, physiological saline, and the like.

The peptide vaccine can be administered via a suitable route of administration in accordance with the type of usage, such as oral administration, intravenous administration, subcutaneous administration, and the like. Examples of the formulation include tablets, granules, soft capsules, hard capsules, liquid, oil solution, emulsion, and the like. The dose of the pharmaceutical composition will vary suitably depending on the condition of the patient to be treated. Generally a preferred dose is 0.1 to 10 mg of a peptide per day for an adult, and the preferred interval is 1 administration every few days to every few months. The peptide vaccine may be used together with an antiviral drug such as interferon and ribavirin.

Before administering the peptide derived from the hepatitis C virus of the present invention, the reactivity of anti-HCV antibodies to the peptide or the presence of CTL can be measured in the blood of a patient. In this case, the term "reactivity of anti-HCV antibodies to the peptide" means that the peptide to be administered is recognized by anti-HCV antibodies already present in the blood of the patient.

Therapeutic efficacy in an HCV-related disease can be verified by monitoring the antibody titer in the blood of the subject afflicted with the HCV-related disease, i.e., measuring the concentration in the blood of antibodies that are reactive with the peptide, by a conventional method such as ELISA. The amount of virus can also be monitored by measuring the amount of HCV RNA by a conventional method such as RT-PCR.

The peptide of the present invention is also useful as a composition for diagnosing a patient having an HCV-related disease or for prognosis of the progression of the disease. Using the peptide of the present invention, the antibody titer in the blood of a subject can be measured using the antigen-antibody reaction that is well known in the art. For example, when a typical ELISA procedure is used, the titer can be measured as follows. The peptide serving as the antigen is attached to a conventional 96-well ELISA plate, and optionally the plate is blocked to prevent nonspecific adsorption. Next serum prepared from the blood of the subject is suitably diluted, added to each well of the plate, and reacted for a predetermined time. The plate is then rinsed, and after the unbound components are removed, an antibody that can bind to a human antibody (for example, a rabbit anti-human antibody) is added. When it is desirable to detect IgG, a y-chain-specific anti-human IgG can be used. After the plate is reacted for a predetermined time, the plate is rinsed, and an antibody (for example, anti-rabbit IgG) that has been detectably labeled is added. Labeling can be performed by a method well known in the art by using an enzyme, fluorescent dye, chemiluminescent substance, biotin and radioactive compound. After the plate is reacted for a predetermined time, a suitable substrate is added, and the decrease in substrate or increase in product is measured, or the label is detected by measuring the fluorescence, luminescence, or radioactivity thereof. In this manner, the amount of antibodies to the specified peptide present in the serum of the subject can be measured. In addition, it is possible to estimate the progression of an HCV-related disease by monitoring the amount of antibodies.

Among the methods for detecting antibodies by such an antigen-antibody reaction, the xMAP technique is available as one method that provides a high level of sensitivity. This is a flowmetry measurement method using a fluorescent microbead array system developed by the Luminex Corporation. Microbeads conjugated with the peptide are brought into direct contact with serum. Then a fluorescently labeled secondary antibody is bound thereto, and the intensity of fluorescence is measured by flowmetry.

When measuring the antibody titer in a hospital laboratory, immunochromatography may be used. In this method an antigen (or antibody) is distributed in lines on a test paper, and a complex of the antibody in the sample and the antigen labeled with colored particles is applied to the test paper. When the complex moves on the test paper, a colored line will appear due to the concentrated capture of the antigen (or antibody). The presence or absence of the colored line provides a qualitative analysis. By this method, results can be obtained with simple equipment in a short time (within 20 to 30 min).

According to the present invention, it is also possible to investigate whether HLA-A24 restricted cytotoxic T lymphocytes have been induced or not in HCV-infected HLA-A24 positive individuals. For example, peripheral blood monocytes (PBMC) are prepared from the HCV-infected individual, and cultured together with the peptide of the present invention. The reactivity of the PBMC to C1R-A24 cells pulsed with the same peptide or an HLA-A24 positive HCV-infected cell (line) may be examined as indicated by IFN-y production or a ⁵¹Cr release reaction. The HLA-Al1, HLA-A31, and HLA-A33 individuals may be tested in the same way. Moreover, it is possible to verify the MHC restriction of the induced CTL by an inhibition assay using MHC class I and CD8 monoclonal antibodies, and peptide specificity can be verified by an inhibition assay using non-labeled target cells sensitized by the corresponding peptide (cold target inhibition test). More specifically, such measurements may be carried out by the methods described in example 1 below.

The entire contents of all patent and reference documents specifically cited in this description are hereby incorporated by reference in its entirety. In addition, the entire contents of the descriptions and drawings of Japanese Patent Applications 2006-14000 and 2006-220542, which serve as the basis for the priority claim of the subject application, are hereby incorporated by reference in its entirety.

The present invention is described in greater detail below through examples. However, these examples are presented merely as illustrations for describing the present invention more specifically, and the present invention is by no means limited to these examples.

### [Example 1]

### Peptide-specific CTL induction and cytotoxic activity of peptide-stimulated PBMC by peptide C35-44 Subjects

The subjects of this assay were HCV-infected individuals with HLA-A24 or HLA-A3 supertype. It was verified that these subjects were not infected by the human immunodeficiency virus (HIV). To obtain the peripheral blood monocytes (PBMC), 20 mL of peripheral blood was collected and the PBMC were prepared by Ficoll-Conley centrifugation. All samples were stored at low temperature until used in the test. The study was approved by The Institutional Ethical Review Board of Kurume University School of Medicine, and informed written consent was obtained from the subjects before the start of the test. Expression of HLA-A11, HLA-A31 and HLA-A33 molecules in the PBMC of the HCV-infected subjects was measured by flow cytometry using the following antibodies: anti-HLA-A11 monoclonal antibody (mAb) (Cat# 0284HA; One Lambda Inc., Canoga, CA), anti-HLA-A31 mAb (Cat# 0273HA, One Lambda), and anti-HLA-A33 mAb (Cat# 0612HA; One Lambda).

### Cell lines

The cell line used was C1R-A24, which is a C1R lymphoma subline expressing HLA-A24 (Dr. M. Takiguchi, Kumamoto University, Japan). The cells were maintained in RPMI 1640 medium supplemented with 10% FCS (fetal calf serum) and 500 µg/mL hygromycin B (Gibco BRL, New York, NY, USA). In addition, cell lines C1R-A11, C1R-A31, and C1R-A33 are C1R lymphoma sublines that were transfected with HLA-A1101, HLA-A3101, and HLA-A3303 genes, respectively, and established to permanently express those antigens. Expression of the HLA-A11, HLA-A31, and HLA-A33 molecules in these sublines has already been reported (Takedatsu H, et al., Clinical cancer Research 2004; 10:1112-20). These cell lines were maintained in RPMI 1640 containing 10% FCS (Invitrogen).

### Peptides

The following peptides were used: peptide C35-44 (YLLPRRGPRL (SEQ ID NO: 1)); as a positive control, a peptide derived from the influenza virus (Flu) having an HLA-A24 binding motif (RFYIQMCTEL (SEQ ID NO: 2)); and as negative controls, a peptide derived from the Epstein-Barr virus (EBV) (TYGPVFMCL (SEQ ID NO: 3)) and a peptide derived from HIV (RYLRDQQLL (SEQ ID NO: 4)). As control peptides that will bind to the HLA-A3 supertype, the following were used: a peptide derived from the influenza virus (Flu) (NVKNLYEKVK (SEQ ID NO: 5)): a peptide derived from the Epstein-Barr virus (EBV) (AVFDRKSDAK (SEQ ID NO: 6)); a peptide derived from tyrosinase-related protein 2 (TRP2) (LLGPGRPYR (SEQ ID NO: 7)); and a peptide derived from HIV (RLRDLLLIVTR (SEQ ID NO: 8)). All the peptides were purchased from BIOSYNTHESIS (Lewisville, TX, USA), SynPep (Dublin, CA, USA), or Biologica Co. (Nagoya, Japan) and the purity was at least 90%. The peptides were dissolved in dimethyl sulfoxide (DMSO) to a concentration of 10 mg/mL and stored at -20°C.

### Statistics

Statistical analysis was performed using the Student t-test. The level of statistical significance was set at P<0.05.

### Detection of peptide-specific IgG

First the peptide-specific IgG level was measured using a 1:100 dilution sample of serum from 12 HCV-infected subjects having HLA-A3 supertype. The peptide-specific IgG level in plasma (or serum) was measured by the Luminex^{™} method following the procedure described above (Komatsu N. et al., Scand J Clin Lab Invest, 64, 535-546, (2004)). Briefly, 200 µL of plasma (1:100 dilution) were combined with 25 µL of peptide-bound color-coded beads (Luminex Corp. (Austin, TX)) in a 96-well filter plate (MABVN1250; Millipore Corp., Bedford, MA) and incubated for 2 hours at room temperature on a plate shaker. After 2 hours, the plate was rinsed in 0.05% Tween 20-PBS, 100 µL of biotinized goat anti-human IgG was added, and incubated for 1 hour at room temperature on a plate shaker. Next, the plate was rinsed, 100 µL of streptavidin-PE (5 µg/mL) was added to the wells, and the mixture was incubated for 30 min at room temperature on a plate shaker. The fluorescence intensity (FI) was measured by Luminex^{™} to determine the level of peptide-specific antibody. Plasma from 15 healthy donors was used as a negative control. The cutoff FI value was set at a value higher than the mean + 2 SD of the FI value in healthy donors.

IgG reactive to the C35-44 peptide was detected in the plasma of 7 out of 12 test subjects. On the other hand, no reactivity was found in the plasma of any of the 15 healthy donors. The mean level of IgG that was reactive to the peptide C35-44 in the test subjects was higher than in each of the test donors, and the differences were all statistically significant.

### Induction of peptide-specific CTL from PBMC of HCV-infected individuals

Next, the peptide C35-44 was tested for its ability to induce peptide-specific CTL from the PBMC of HCV-infected individuals of HLA-A24, HLA-A11, HLA-A31, or HLA-A33. After the PBMC were cultured *in vitro* with the HCV-derived peptide or a control peptide, the reactivity of the PBMC to C1R-A24, C1R-A11, C1R-A31, or C1R-A33 cells pulsed with the corresponding peptide was examined as indicated by IFN-Υ production.

The assay to detect the peptide-specific CTL followed the method previously reported with slight modifications (Hida N et al., Cancer Immunol Immunother 2002; 51: 219-28). Briefly, PBMC (1 × 10⁵ cells/well) from HLA-A24-positive HCV-infected subjects were incubated together with 10 µL/mL of each peptide in 200 µL of liquid culture medium in a U-bottom 96-well microculture plate (Nunc, Roskilde, Denmark). The test was conducted in quadruplicate. The liquid culture medium comprised 45% RPMI 1640, 45% AIM-V medium (Gibco-BRL, Gaithersburg, MD, USA), 10% FCS, 100 U/mL interleukin-2 (IL-2), and 0.1 mM MEM nonessential amino acid solution (Gibco-BRL). Half of the liquid culture medium was removed every 3 days and replaced with fresh medium containing the peptide (20 µg/mL). On day 15 of culturing, half the cultured cells were stimulated with C1R-A24 cells that had been pulsed with the peptide C35-44, and the other half were stimulated with C1R-A24 cells that had been pulsed with the control HIV peptide. After 18 hours of incubation, the supernatant was collected and the level of IFN-Υ was measured by ELISA.

When the peptide C35-44 was used, CTL reactive to the peptide were induced in the PBMC from 3 out of 5 HLA-A24-positive HCV-infected subjects (Table 1). In the table, the numbers represent the level of IFN-Υ (ng/mL).

**[Table 1]**

| Subject No. | #31 | #32 | #33 | #2 | #34 | |
|---|---|---|---|---|---|---|
| HLA | A24 | A24 | A24 | A24/A11 | A24/A2 | |
| C35-44 | 1723 | 0 | 2117 | 0 | 714 | 3/5 |
| EBV | 440 | 0 | 531 | 0 | 0 | 2/5 |
| Flu | 222 | 1014 | 0 | 0 | 661 | 3/5 |

Next, the peptide C35-44 was tested for its ability to induce HLA-A3 supertype-restricted CTL. PBMC from HLA-A2 or HLA-A3 supertype-positive HCV-infected individuals were stimulated *in vitro* with the peptide C35-44 or a control peptide, and IFN-Υ production was measured in T2 (having HLA-A2), C1R-A11, C1R-A31 and C1R-A33 cells pulsed with the corresponding peptide. As a result, the peptide C35-44 induced peptide-reactive CTL in PBMC from HLA-A11-positive HCV-infected individuals (0/7), HLA-A31-positive HCV-infected individuals (1/2), and HLA-A33-positive HCV-infected individuals (1/2).

These findings show that the peptide C35-44 can induce peptide-specific CTL in PBMC from HCV-infected individuals with HLA-A11, HLA-A31, or HLA-A33.

It was already known that the HCV peptide C35-44 can induce HLA-A2-restricted CTL (Takao Y et al., Microbiol. Immunol., 48(7), 507-517, 2004; Japanese Patent Application 2005-310203). The above results demonstrate that the peptide C35-44 can also induce CTL from HLA-A24, HLA-A11, HLA-A31, or HLA-A33-positive HCV-infected individuals, despite the fact that it exhibits extremely low binding activity with HLA-A24, HLA-A11, HLA-A31, and HLA-A33 when the dissociation constants are calculated by the conventional method.

### Cytotoxic activity of peptide-stimulated PBMC

The cytotoxic activity of peptide-stimulated PBMC toward C1R-A24, -A11, -A31, or -A33 cells using a standard 6 hour ⁵¹Cr release assay. The cells had been pulsed preliminarily with either of the corresponding peptide or an HIV peptide. Two thousand ⁵¹Cr-labeled cells per well were cultured together with effector cells in a round-bottom 96-well plate at a predetermined effector cell/labeled cell ratio. Specific ⁵¹Cr release was calculated by the formula: (test group c.p.m. - natural release c.p.m.). Natural release was determined from the supernatant of labeled cells incubated without effector cells. Total release was determined from the supernatant of labeled cells incubated with 1% Triton-X (Wako Pure Chemical Industries, Osaka, Japan). In some cases, 10 µg/mL of anti-HLA-class I (W6/32: mouse IgG2a), anti-HLA-DR (L243: mouse IgG2a), anti-CD4 (NU-TH/I: mouse IgG1), anti-CD8 (NU-TS/C: mouse IgG2a), or anti-CD14 (H14: mouse IgG2a) monoclonal antibody was added to the wells at the start of culturing.

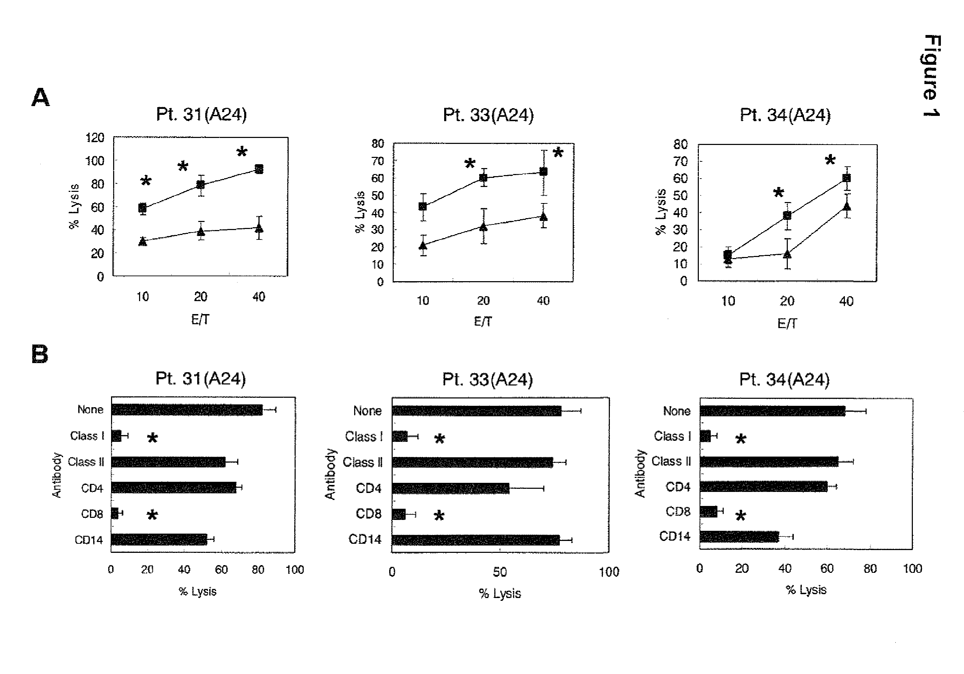

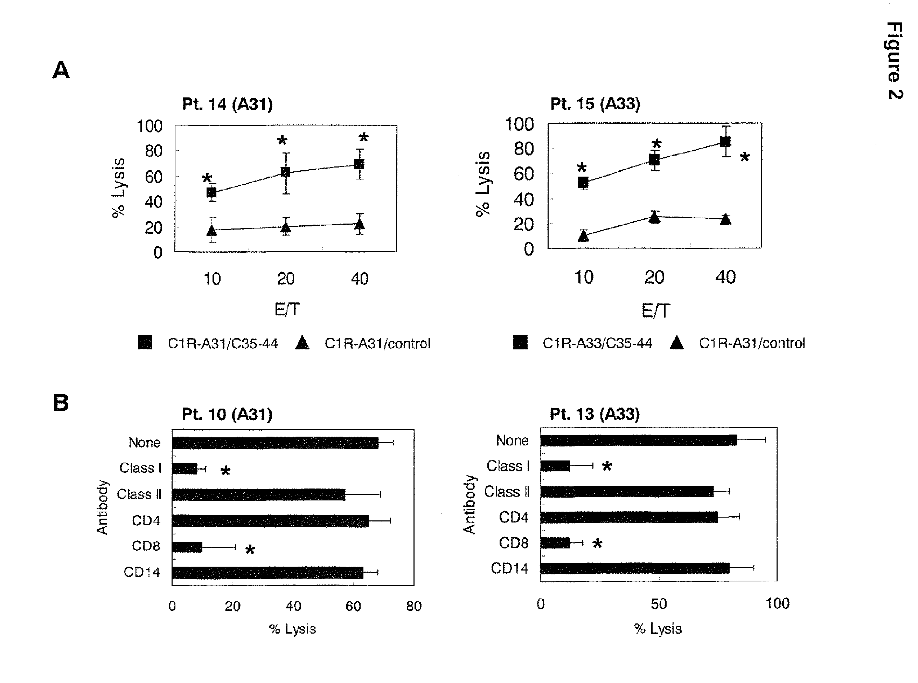

The PBMC from HLA-A24, HLA-A11, HLA-A31, or HLA-A33-positive HCV-infected individuals cultured together with the HCV peptide C35-44 exhibited cytotoxic activity toward C1R-A24, -A11, -A31, or -A33 cells preliminarily loaded with this peptide, but exhibited no cytotoxic activity with the HIV peptide (FIGS. 1 and 2).

In addition, cytotoxic activity toward C1R-A24, -A11, -A31, and -A33 cells pulsed with the peptide C35-44 was inhibited by HLA-class I (W6/32) or CD8 monoclonal antibodies (mAb), while it was not inhibited by HLA-class II (DR-1), CD4, or CD14 mAb tested. This finding indicates that the peptide-specific cytotoxic activity is mainly expressed through CD8⁺ T cells in a HLA-class I restricted manner (FIGS. 1 and 2).

The above results demonstrate that the peptide C35-44 is useful for peptide immunotherapy in HLA-A24, HLA-A11, HLA-A31, or HLA-A33 positive patients afflicted by HCV-related diseases.

### [Example 2]

### Binding assay of peptide C35-44 with HLA-A24, HLA-11, HLA-A31, and HLA-A33

The peptide C35-44 was assayed for its ability to bind to HLA-A24, HLA-A11, HLA-A31, and HLA-A33 by a direct contact assay using MHC molecules.

### Cell lines

The RMA cell line is a lymphoma cell line transformed with Rauscher virus derived from C57BL/6 (H-2b), and RMA-S is a TAP-deficient natural mutation cell line (Ljunggren HG, and Karre K: J Exp Med, 162, 1745, 1985). RMA-S-A*2402/Kb is a mouse lymphoma cell line wherein the -A*2402/Kb chimeric gene encoding the a1 and a2 domains of the HLA-A*2402 gene, and the a3, transmembrane, and intracellular domains from the murine H-2kb molecule, has been introduced into RMA-S cells (provided by Dr. Takasu (Research Institute of Dainippon Sumitomo Pharma Co., Ltd., Osaka, Japan)). On the other hand, RMA-S-A*0301, - A*1101, -A*3101, and -A*3303 cell lines were established by introducing the human HLA-A*0301, -A*1101, -A*3101, and - A*3303 genes, respectively, into murine TAP-1-deficient RMA-S cells.

### Test method

Because RMA-S-A*2402/Kb and the RMA-S-A*0301, -A*1101, -A*3101, and -A*3303 cell lines are TAP-deficient cell lines, they cannot form a complex with a peptide. Therefore, MHC molecules cannot be present stably on the cell surface under normal culturing conditions. Taking advantage of this property, the peptide C35-44 was tested for its ability to bind to HLA-A24, HLA-A11, HLA-A31, and HLA-A33. Briefly, the peptide C35-44 was added to RMA-S-A*2402/Kb or RMA-S-A*0301, - A*1101, -A*3101, and -A*3303 cell lines cultured at 26°C (under these conditions, MHC molecules can exist on the cell membrane), and flow cytometry was used to measure the extent that the MHC molecules on the cell surface disappeared when the cells were cultured at 37°C (Townsend A, et a., Cold Spring Harb Symp Quant Biol, 1, 299-308, 1989).
More specifically, the test was conducted using the following procedure.
1. Culture RMA-S-A*2402/Kb overnight (18 to 20 hours) at 26° C
2. Rinse once with PBS
3. Suspend the cells (2 × 10⁵) in OPTI-MEM (Life Technologies) containing 3 µg/mL ß2-microglobulin (Cortex Biochem, San Leandro, CA, USA) and a peptide (100 µg/mL)
4. Culture 3 hours at 26°C
5. Culture 3 hours at 37°C
6. Rinse once with PBS containing 3% FCS
7. Incubate 30 min at 4°C with anti-HLA-mAb (primary antibody)
8. Rinse once with PBS containing 3% FCS
9. Incubate for 30 min at 4°C with PE (phycoerythrin: a phycobiliprotein that excites effectively at 488 nm and emits fluorescence at 578 nm)-rabbit anti-mouse IgG (secondary antibody)
10. Rinse once with PBS containing 3% FCS
11. Suspend cells in 1 mL PBS containing 1% formaldehyde
12. Measure with EPICS (flow cytometer, Beckman Coulter)

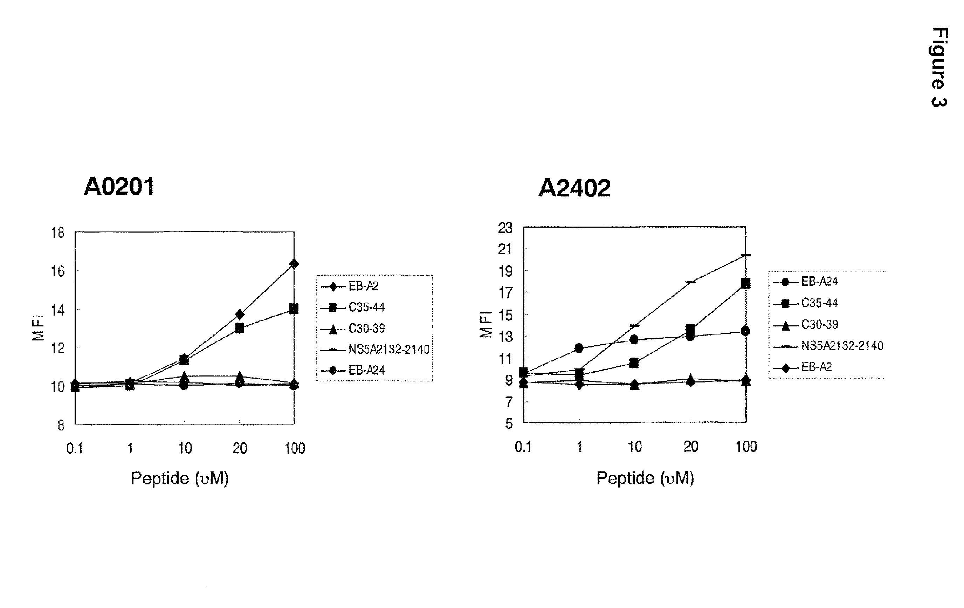

The results are shown in FIG. 3. After the RMA-S-A*2402/Kb or RMA-S-A*0201 cells were cultured at 26° C, the mean channel (X-mean) was 10 for each cell line when cultured at 37°C without a peptide pulse. After the RMA-S-A*2402/Kb cells were cultured at 26°C, then pulsed with the peptide C35-44 at concentrations ranging from 0.1 to 100 µM, and cultured at 37°C, the X-mean increased in a concentration dependent manner (FIG. 3). Under the same conditions, EB-A2 having the HLA-A2 binding motif, the peptides NS5A-2132 and EB-A24 having the HLA-A24 binding motif were tested as positive controls, and binding was observed in both cases. On the other hand, no binding was seen with any of the peptides used as negative controls (C30, NS5A2132, and EB-A24 were used for HLA-A2, and EB-A2 and C30 were used for HLA-24.)

In summary, it was found that the peptide C35-44 can bind to both HLA-A2 and -A24 as indicated by increase in the fluorescence intensity in a concentration dependent manner with respect to the pulsed peptide, in the same manner as in the positive controls.

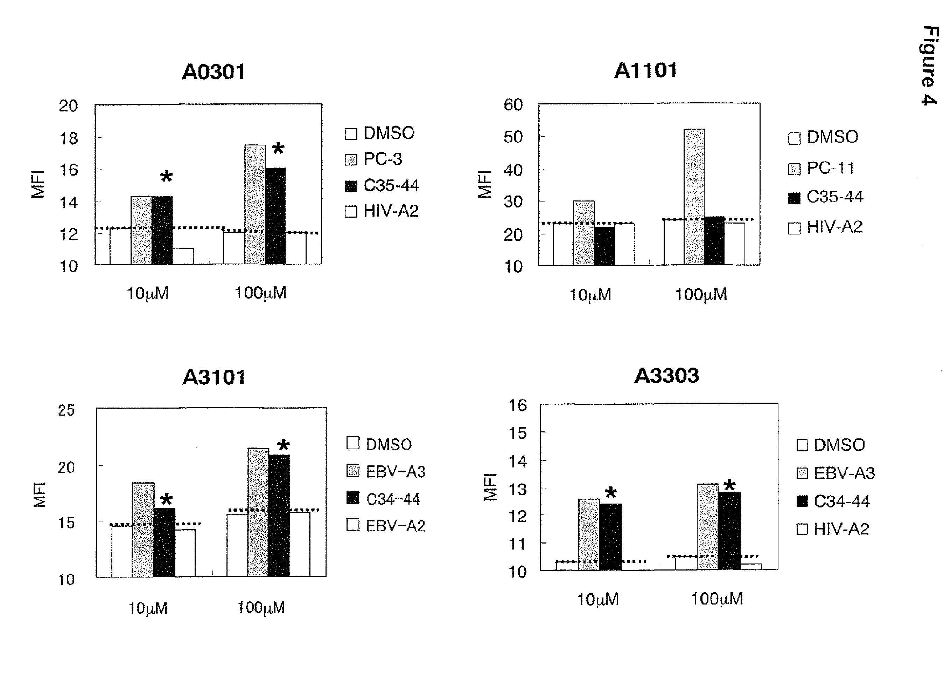

The peptide binding capability toward HLA-A3 supertype was investigated in the same manner. As a result, it was found that the HCV peptide C35-44 binds to HLA-A3, -A31, and - A33 at about the same extent as the positive control peptides, while it did not bind to HLA-A11 (FIG. 4).

### [Example 3]

### HLA-A11 restricted CTL induction from peripheral blood from healthy donors by peptide C35-44

The peptide C35-44 was tested for its ability to induce peptide-specific CTL in PBMC from healthy donors having HLA-A11. After the PBMC were incubated *in vitro* with the HCV-derived peptide or a control peptide, their reactivity toward C1R-A11 cells pulsed with the corresponding peptide was measured as indicated by IFN-Υ production.

Lymphocytes from six healthy donors (HD) [HLA-A11/A11, A2/A2, A24/A24, A2/A24, A11/A2, and A11/A24] were used for CTL induction. The peptide-specific CTL was measured according to a previously reported method with slight modifications (Hida N et al., Cancer Immunol Immunother 2002; 51: 219-28). Briefly, PBMC (1 × 10⁵ cells/well) from HLA-A11 positive healthy donors were incubated together with 10 µL/mL of each peptide in 200 µL of liquid culture medium in a U-bottom 96-well microculture plate (Nunc, Roskilde, Denmark). The test was conducted in quadruplicate. The liquid culture medium comprised 45% RPMI 1640, 45% AIM-V medium (Gibco-BRL, Gaithersburg, MD, USA), 10% FCS, 100 U/mL interleukin-2 (IL-2), and 0.1 mM MEM nonessential amino acid solution (Gibco-BRL). Half of the liquid culture medium was removed every 3 days and replaced with fresh medium containing the peptide (20 µg/mL). On day 15 of culturing, half the cultured cells were stimulated with C1R-A11 cells that had been pulsed with the peptide C35-44, and the other half were stimulated with C1R-A11 cells that had been pulsed with the control HIV peptide. After 18 hours of incubation, the supernatant was collected and the level of IFN-Υ was measured by ELISA.

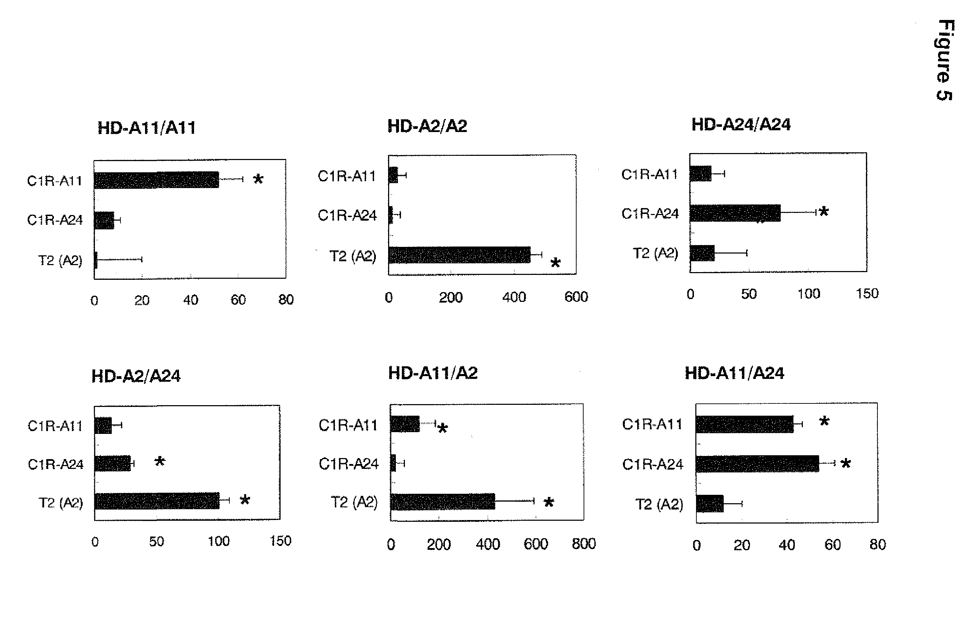

The results are shown in FIG. 5. When the peptide C35-44 was used, peptide-reactive CTL were induced in the PBMC from 3 out of 3 HLA-A11-positive healthy donors. In the graphs, the numbers on the horizontal axis represent the level of IFN-γ (ng/mL). The above results demonstrate that the peptide C35-44 induces HLA-A11-restricted CTL.

### INDUSTRIAL APPLICABILITY

The peptide of the present invention is useful in the treatment of HCV-related diseases.

## Claims

1. A pharmaceutical composition for the treatment of an HCV-related disease in a patient having HLA-A24 or -A3 supertype, comprising a peptide having the amino acid sequence: (YLLPRRGPRL (SEQ ID NO: 1) as an active ingredient.

2. A composition for detecting the induction of an HLA-A24 or -A3 supertype-restricted cytotoxic T cell in a patient having HLA-A24 or -A3 supertype, comprising a peptide having the amino acid sequence: YLLPRRGPRL (SEQ ID NO: 1) as an active ingredient.
